# EUROPEAN PATENT APPLICATION

(11) **EP 2 343 058 A2**
(43) Date of publication of application: **13.07.2011**
(21) Application number: 11157129.5
(22) Date of filing: 27.10.2006
(51) Int. Cl.: A61K 9/20

(54) **A pharmaceutical formulation containing olanzapine**

(30) Priority: 03.11.2005 GB 0522473
(62) Divisional of application: 06842365.6
(71) Applicant: Actavis Group PTC ehf., 220 Hafnarfjordur (IS)
(72) Inventor: Stefansson, Stefan, Einar, 220 Hafnarfjordur (IS)
(74) Representative: Cooke, Richard Spencer

(57) **Abstract**

This invention relates to a stable pharmaceutical formulation containing olanzapine. The composition comprises olanzapine or a pharmaceutically acceptable salt thereof, one or more suitable pharmaceutical excipients and a compound of formula I and/or a compound of formula II: 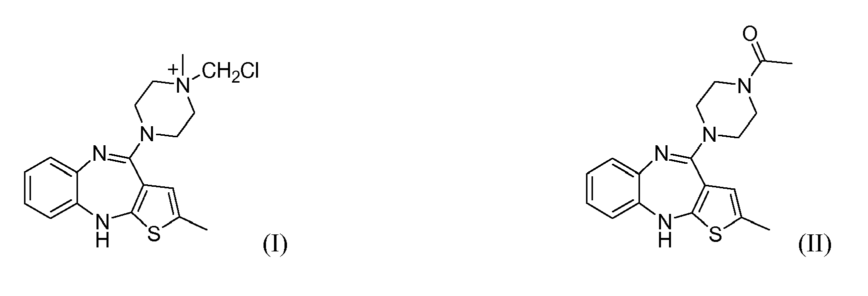 or a pharmaceutically acceptable salt thereof.

## Description

This invention relates to a pharmaceutical formulation and in particular to a stable composition for a pharmaceutical dosage form containing olanzapine.

Olanzapine (2-methyl-4-(4-methyl-1-piperazinyl)-10H-thieno[2,3-b] [1,5] benzodiazepine) is a psychotropic agent belonging to the class of drugs known as thienobenzodiazepines. Olanzapine is used for the treatment of schizophrenia and is indicated for the prevention of recurrence of manic episodes in patients with bipolar disorder whose manic episode has responded to olanzapine treatment.

US 4,115,568 discloses a general formula of thieno[1,5] benzodiazepines having useful CNS activity. US 5,229,382 discloses olanzapine per se as well its CNS activity.

However, the current commercially available tablets are limited by a high rate of degradation of the drug. Olanzapine is known to suffer from problems associated with the intrinsic nature of olanzapine, e.g. moisture sensitivity, propensity for discoloration, metastability of various crystalline and amorphous forms and degradation after compounding into tablets.

A number of attempts have been made to avoid the degradation of olanzapine. For example, WO 2005/009407 discloses a pharmaceutical composition containing olanzapine particles or powder in which the olanzapine particles or powder have a coating comprising lactose and/or mannitol. This document also discloses a pharmaceutical composition containing olanzapine and suitable excipients in which the olanzapine and excipients have a coating selected from carrageenan, sodium alginate, polyvinyl alcohol-polyethylene glycol graft copolymer, and a titanium dioxide-talc mixture.

There remains, however, a need to provide further and/or improved formulations to avoid degradation and discolouration of olanzapine formulations.

Accordingly, the present invention provides a pharmaceutical composition comprising olanzapine or a pharmaceutically acceptable salt thereof, one or more suitable pharmaceutical excipients and a compound of formula I and/or a compound of formula II: 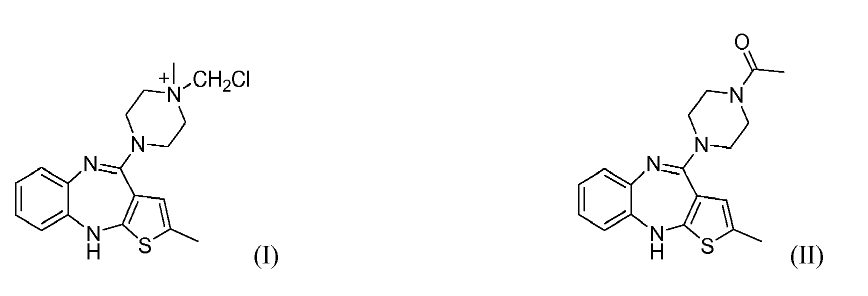 or a pharmaceutically acceptable salt thereof.

The present invention further provides a compound of formula I or a pharmaceutically acceptable salt thereof and a compound of formula II or a pharmaceutically acceptable salt thereof.

Olanzapine, although successfully used for the treatment of schizophrenia, has several intrinsic properties which need to be addressed when developing a composition to be compounded into a tablet. The active ingredient has propensities to degrade after compounding into a tablet, discolour, transform into non-desirable polymorphic forms, and finally it is also known to be moisture sensitive.

Furthermore, any impurities, e.g. impurities which are associated with either excipients or the active compound, can potentially, either by themselves or in synergy, further augment the instability of the drug. These impurities may, for example, catalyse a chemical degradation, act as a seed for uncalled crystal transformation or be hygroscopic in nature, and therefore be catastrophic as an ingredient in the compounded tablets.

In addition, the discoloration of olanzapine and a mottled appearance of tablets, although not accompanied with degradation of olanzapine, are not considered to be pharmaceutically acceptable.

It has been found, surprisingly, that incorporating a compound of formula I and/or a compound of formula II into a pharmaceutical composition containing olanzapine reduces the rate of degradation of olanzapine when compounded into tablets, provides stable crystal forms of olanzapine, prevents discoloration and also eliminates the moisture sensitivity of the drug. The compounds of formula I and of formula II have also been found to be pharmaceutically active.

In an embodiment of the present invention, a pharmaceutical composition containing olanzapine or a pharmaceutically acceptable salt further comprises a compound of formula I or a pharmaceutically acceptable salt thereof. The compound of formula I or the salt is preferably present at a minimum of 0.001, more preferably 0.01 and most preferably 0.05 wt%, and at a maximum of 5, more preferably 1, more preferably 0.5 and most preferably 0.1 wt% based on the weight of olanzapine or the pharmaceutically acceptable salt thereof.

In another embodiment of the present invention, a pharmaceutical composition containing olanzapine or a pharmaceutically acceptable salt further comprises a compound of formula II or a pharmaceutically acceptable salt thereof. The compound of formula II or the salt is preferably present at a minimum of 0.001, more preferably 0.01 and most preferably 0.05 wt%, and at a maximum of 5, more preferably 1, more preferably 0.5 and most preferably 0.1 wt% based on the weight of olanzapine or the pharmaceutically acceptable salt thereof.

In a further embodiment, a pharmaceutical composition containing olanzapine or a pharmaceutically acceptable salt further comprises a compound of formula I and a compound of formula II or a pharmaceutically acceptable salt of either compound. The compound of formula I and the compound of formula II or their salts are preferably present at a minimum of 0.001, more preferably 0.01 and most preferably 0.05 wt%, and at a maximum of 5, more preferably 1, more preferably 0.5 and most preferably 0.1 wt% based on the weight of olanzapine or the pharmaceutically acceptable salt thereof. The weight percent values relate to the combination of the compound of formula I and formula II. The ratio of the compound of formula I and formula II may be from 95:5 to 80:20, more preferably 90:10 to 85:15 and most preferably about 90:10.

Olanzapine is a known compound and may be synthesised using the procedure disclosed in US 5,229,382.

The compound of formula I (2-methyl-4-(4-methyl-4-chloromethyl-1-piperazinyl)-10H-thieno-[2,3-b][1,5]-benzodiazepine): 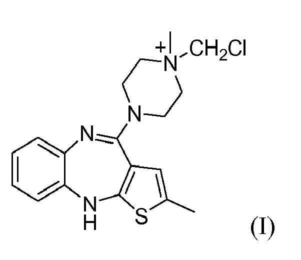 may be synthesised by the following route:

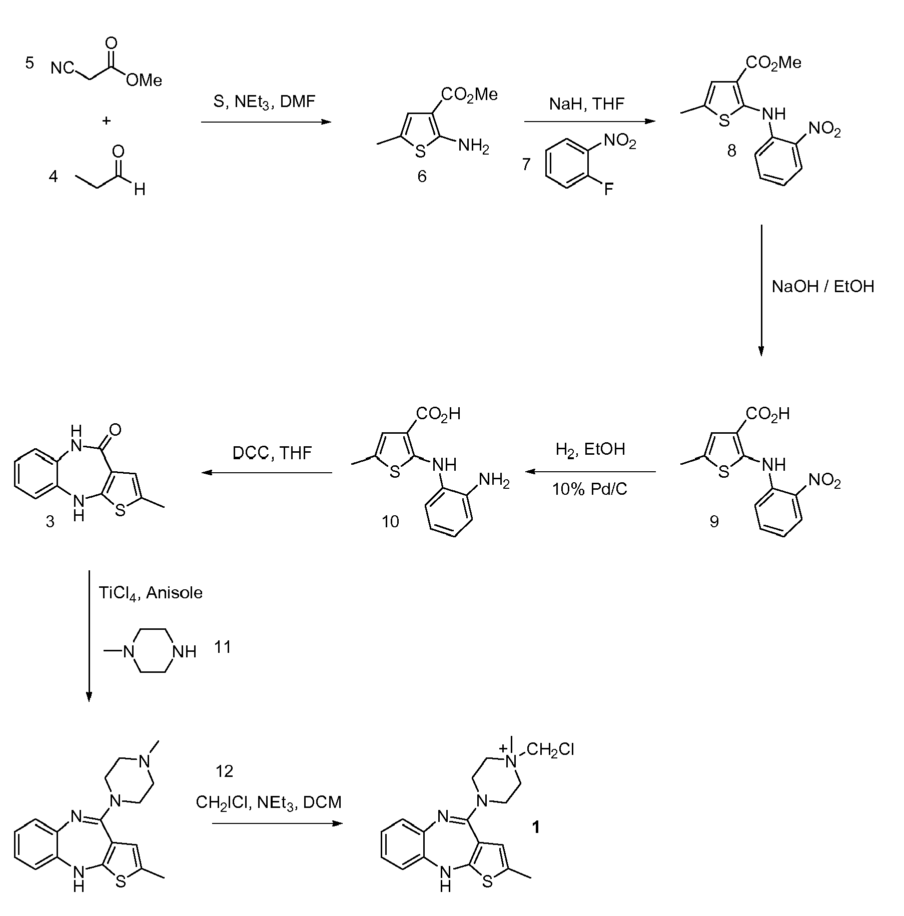

The compound of formula II (2-methyl-4-(4-acetyl-l-piperazmyl)-10H-thieno-[2,3-b][l,5]-benzodiazepine): 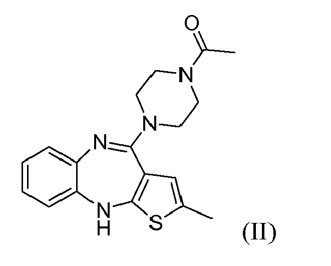 may be synthesised by the following route:

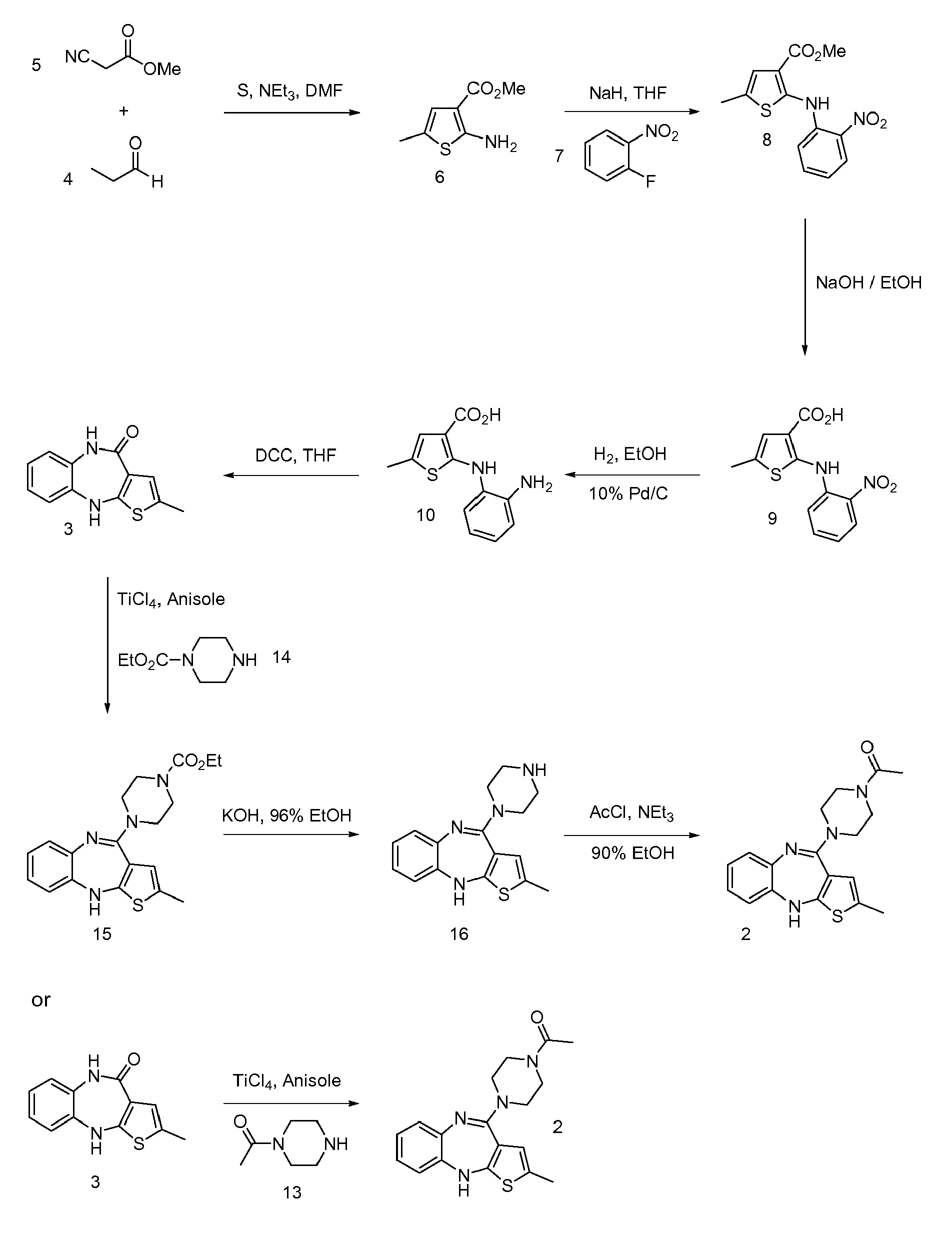

Both of the compounds of formula I and formula II may be made via a common intermediate lactam 3. This known compound, see US 5,229,382, may be synthesised in five steps starting with condensation of propionaldehyde 4, sulfur and malonitrile 5 to produce thiophene 6; nucleophilic substitution of 2-fluoronitrobenzene 7 by deprotonated thiophene 6; alkaline hydrolysis of the product ester 8 to give acid 9; hydrogenation of the nitro group of acid 9 to give amino acid 10; and then a lactam formation promoted by a coupling reagent, such as DCC, to give lactam 3.

TiCl₄-promoted addition-substitution of N-methylpiperazine 11 to the lactam 3 and then addition of an appropriate electrophile, such as chloroiodomethane 12, gives the compound of formula I (numbered 1 in the scheme). Other one-carbon bis-electrophiles could also be used in place of chloroiodomethane. These one-carbon bis-electrophiles require the presence of a methylene unit attached to a leaving group and an atom, or group of atoms, which is or could be converted to chlorine. Such one-carbon bis-electrophiles are known in the art and are exemplified by CH₂Cl₂, CH₂BrCl and iodomethane methyl ether.

Similarly, direct reaction of commercially available piperazine 13 in the presence of TiCl₄ gives the compound of formula II (numbered 2 in the scheme). Alternatively, the compound of formula II may be synthesised in three steps, based on the procedure disclosed in US 4,115,568. Addition-substitution of lactone 3 by ethyl 1-piperazinecarboxylate 14 in the presence of TiC4 followed by hydrolysis-decarboxylation of the carbamate product 15 gives amine product 16 which is acylated by an acylating agent, such as acyl chloride, in the presence of NEt₃ to give the compound of formula II.

Consecutive addition, in either order, of a one-carbon bis-electrophile and a suitable electrophilic methyl equivalent to amine product 16 offers an alternative route to the synthesis of the compound of formula I.

A complementary approach to the synthesis of the compound of formula I and the compound of formula II is via the thioamide analogous to lactam 3. This may be synthesised by aromatic nucleophilic substitution of 1-flouro-2-nitro-benzene with 3-cyano-4-aminothiophene. Reduction of the nitro group by hydrogenation would then produce an amine which would undergo ring closure in the presence of acid catalysis onto the cyano group to giving an amidine. This intermediate amidine may also be synthesised starting with condensation of 3-cyano-4-oxotetrahydrothiophene and 1-amino-2-nitrobenzene to give an enamine, which is aromatised using chloranil to give a thiophene. The nitro group of this thiophene is then reduced to an amine, which undergoes ring closure in the presence of acid catalysis onto the cyano group to give an amidine. Treatment of this amidine produced by either route with PS₅ in anhydrous pyridine gives the required thioester. Due to the higher relative reactivity of the thioester in comparison to the lactam 3, the thioester reacts with piperazines 11, 13 and 14 directly without TiCl₄ catalysis to give either intermediates which can be converted using previously described routes into the compound of formula I or the compound of formula II, or these compounds directly.

As well as the free base form, olanzapine and the compounds of formula I and formula II may also be used as pharmaceutically acceptable acid addition salt forms. Such salts include salts of inorganic acids, such as hydrobromic, hydrochloric, nitric, phosphoric or sulfuric acids; and salts of organic acids, such as organic carboxylic acids, for example citric, fumaric, glycollic, hydroxymaleic, lactic, maleic, malic, or tartaric acids, or organic sulfonic acids, for example ethane sulfonic, 2-hydroxyethane sulfonic, methane sulfonic, naphthalene-2-sulfonic or toluene-p-sulfonic acids.

The compounds of formula I and II, as well as being useful in stabilising a formulation containing olanzapine, also show central nervous system (CNS) activity, as may be demonstrated using known models for CNS disorders.

Accordingly, the present invention also provides a pharmaceutical composition comprising the compound of formula I or a pharmaceutically acceptable salt thereof and/or formula II or a pharmaceutically acceptable salt thereof, and one or more suitable pharmaceutical excipients. The present invention also provides a method of treating schizophrenia and for the prevention of recurrence of manic episodes in patients with bipolar disorder whose manic episode has responded to olanzapine treatment or treatment with an olanzapine derivative, such as a compound of formula I or formula II.

The compounds of formula I and formula II show antagonised apomorphine-induced climbing behaviour and hypothermia in mice (Moore, N. A. et al. Psychopharmacology 94 (2), 263-266 (1988), and 96, 539 (1988)) at doses of less than 10 mg/kg. These compounds also inhibit a conditioned avoidance response in rats at a low dose (ED₅₀ about 5 mg/kg) without inducing catalepsy.

In addition, the compound of formula I and of formula II also show good activity in in vitro binding assays involving binding to neural receptors. The compounds are active at both the dopamine D-1 and D-2 receptors as indicated by an IC₅₀ of less than 1 µM in the ³H-SCH23390 (Billard, W. et al. Life Sciences 35 1885 (1984)) and the ³H-spiperone (Seeman, P. et al. Nature 261 717 (1976)) binding assays. Similar results are found in the ³H-QNB binding assay (Yamamura, HI and Snyder, SH in Proc. Nat. Acad. Sci. USA 71 1725 (1974)) showing antimuscarinic-anticholinergic activity and at the 5-HT-2 receptor showing displacement of H-spiperone from binding sites in the rat frontal cortex (Peroutka, SJ and Snyder, SH Mol. Pharmacol. 16 687 (1979)) at low nanomolar concentrations. The compounds are also active at the 5-HT-IC receptor.

These activity profiles indicate that the compounds are effective in the treatment of psychotic conditions, but are less likely to induce extra pyramidal side-effects.

Olanzapine formulations, or formulations containing the compound of formula I or the compound of formula II, or combinations thereof may be formulated in various dosage forms containing 2 to 20 mg, preferably 2.5, 5, 7.5, 10 or 15 mg of active ingredient.

The formulations contain pharmaceutically acceptable excipients which are well-known in the art. Suitable excipients include binders, disintegrants, fillers and lubricants.

### Examples

### Example 1

| Ingredient | mg per tablet |
|---|---|
| Olanzapine* | 2.5 |
| Lactose anhydrous | 58.3 |
| Microcrystalline cellulose | 16.0 |
| Crospovidone | 2.4 |
| Magnesium stearate non bovine | 0.8 |
| | 80.0 |

| | |
|---|---|
| *The compound of formula I is 0.05% w/w of olanzapine. | |

### Example 2

| Ingredient | mg per tablet |
|---|---|
| Olanzapine* | 2.5 |
| Compactrol | 68.5 |
| Microcrystalline cellulose | 25.0 |
| Crospovidone | 3.0 |
| Magnesium stearate non bovine | 1.0 |
| | 100.0 |

| | |
|---|---|
| *The compound of formula I is 0.05% w/w of olanzapine. | |

### Example 3

Impurities were measured after storage of the tablets from Examples 1 and 2 in aluminium/aluminium blister packs kept at 40°C and 75% relative humidity.

| | % Impurities | | |
|---|---|---|---|
| Time | Tablets from Ex. 1 | Tablets from Ex. 2 | Zyprexa** |
| 0 time point | 0.077 | 0.086 | 0.082 |
| 1 month - blister (alu/alu) | 0.183 | 0.129 | 0.207 |
| 3 month - blister (alu/alu) | 0.4 | 0.2 | 0.65 |

| | | | |
|---|---|---|---|
| **Commercially available tablet containing olanzapine | | | |

The total impurity content of the tablet after prolonged storage is reduced by incorporation of 0.05 wt%, based on the total amount of olanzapine, of the compound of formula I.

### Example 4

Repetition of the above examples using tablets containing the same amount of the compound of formula II shows analogous results.

## Claims

1. A pharmaceutical composition comprising olanzapine or a pharmaceutically acceptable salt thereof, one or more suitable pharmaceutical excipients and a compound of formula I and/or a compound of formula II: 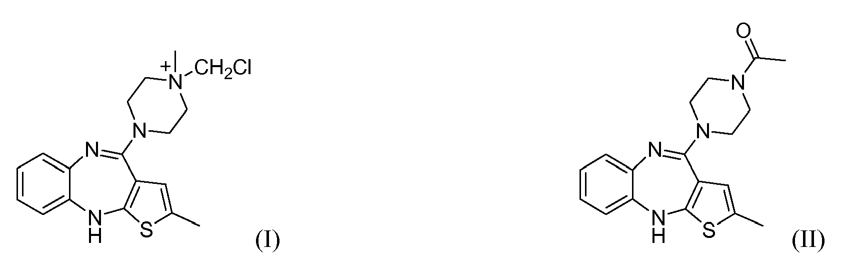 or a pharmaceutically acceptable salt thereof.

2. A pharmaceutical composition according to claim 1, wherein the compound of formula I or a pharmaceutically acceptable salt thereof is present at 0.001 to 5 wt% based on the weight of olanzapine or the pharmaceutically acceptable salt thereof.

3. A pharmaceutical composition according to claim 1, wherein the compound of formula II or a pharmaceutically acceptable salt thereof is present at 0.001 to 5 wt% based on the weight of olanzapine or the pharmaceutically acceptable salt thereof.

4. A pharmaceutical composition according to claim 1, wherein the compound of formula I and the compound of formula II or a pharmaceutically acceptable salt thereof are both present at a total of 0.001 to 5 wt% based on the weight of olanzapine or the pharmaceutically acceptable salt thereof.

5. A pharmaceutical composition comprising the compound of formula I or a pharmaceutically acceptable salt thereof and/or formula II or a pharmaceutically acceptable salt thereof, and one or more suitable pharmaceutical excipients.
